# EUROPEAN PATENT SPECIFICATION

(11) **EP 2 641 591 B1**
(45) Date of publication and mention of the grant of the patent: **21.06.2017**
(21) Application number: 13160042.1
(22) Date of filing: 19.03.2013
(51) Int. Cl.: A61K 9/20, C08B 31/00, A61K 9/00, A61K 47/36, A61K 31/57

(54) **Use of Starch Based Bioadhesive Polymers and Applications in Drug Carrier Systems**
Verwendung von stärkebasierten bioadhäsiven Polymeren und Anwendungen in Arzneimittelträgersystemen
Utilisation de polymères bioadhésifs à base d'amidon et applications dans des systèmes de support de médicament

(30) Priority: 20.03.2012 TR 201203191
(43) Date of publication of application: 25.09.2013
(73) Proprietor: T.C. Istanbul Üniversitesi Rektörlügü Bilimsel Arastirma Projeleri Koordinasyon Birimi, 34452 Istanbul (TR); Özgümüs Pabuccuoglu, Saadet K., 34320 Istanbul (TR); Gök, Mehmet Koray, 34320 Istanbul (TR); Demir, Kamber, 34320 Istanbul (TR); Cirit, Ümüt, 21280 Diyarbakir (TR); Cevher, Erdal, 34116 Istanbul (TR); Özsoy Erginer, Yildiz, 34116 Istanbul (TR); Bacinoglu, Süleyman, 34896 Istanbul (TR); Pabuccuoglu, Serhat, 34320 Istanbul (TR)
(72) Inventor: ÖZGÜMÜS PABUCCUOGLU, SAADET K., 34320 Istanbul (TR); GÖK, MEHMET KORAY, 34320 Istanbul (TR); DEMIR, KAMBER, 34320 Istanbul (TR); CIRIT, ÜMÜT, 21280 Diyarbakir (TR); CEVHER, ERDAL, 34116 Istanbul (TR); ÖZSOY ERGINER, YILDIZ, 34116 Istanbul (TR); BACINOGLU, SÜLEYMAN, 34896 Istanbul (TR); PABUCCUOGLU, SERHAT, 34320 Istanbul (TR)
(74) Representative: Mutlu, Aydin

(56) References cited:
- AMEYE D ET AL: "Ex vivo bioadhesion and in vivo testosterone bioavailability study of different bioadhesive formulations based on starch-g-poly(acrylic acid) copolymers and starch/poly(acrylic acid) mixtures", JOURNAL OF CONTROLLED RELEASE, ELSEVIER, AMSTERDAM, NL, vol. 79, no. 1-3, 19 February 2002 (2002-02-19), pages 173-182, XP004340923, ISSN: 0168-3659, DOI: 10.1016/S0168-3659(01)00539-9
- TRIMNELL D ET AL: "Graft Copolymers from Thiolated Starch and Vinyl Monomers", JOURNAL OF APPLIED POLYMER SCIENCE, JOHN WILEY & SONS, INC, US, vol. 21, no. 3, 1 March 1977 (1977-03-01), pages 655-663, XP009169758, ISSN: 0021-8995 [retrieved on 2003-03-09]
- ATHAWALE V D ET AL: "Graft copolymerization onto starch. II. Grafting of acrylic acid and preparation of it's hydrogels", CARBOHYDRATE POLYMERS, APPLIED SCIENCE PUBLISHERS, LTD. BARKING, GB, vol. 35, no. 1-2, 2 January 1998 (1998-01-02), pages 21-27, XP004126949, ISSN: 0144-8617, DOI: 10.1016/S0144-8617(97)00138-0
- BERNKOP-SCHNURCH A ET AL: "Preparation and characterisation of thiolated poly(methacrylic acid)-starch compositions", EUROPEAN JOURNAL OF PHARMACEUTICS AND BIOPHARMACEUTICS, ELSEVIER SCIENCE PUBLISHERS B.V., AMSTERDAM, NL, vol. 57, no. 2, 1 March 2004 (2004-03-01), pages 219-224, XP027139978, ISSN: 0939-6411 [retrieved on 2004-02-24]
- KAST C E ET AL: "Thiolated polymers - thiomers: development and in vitro evaluation of chitosan-thioglycolic acid conjugates", BIOMATERIALS, ELSEVIER SCIENCE PUBLISHERS BV., BARKING, GB, vol. 22, no. 17, 1 September 2001 (2001-09-01), pages 2345-2352, XP004255097, ISSN: 0142-9612, DOI: 10.1016/S0142-9612(00)00421-X

## Description

### Technical Field of the Invention

The present invention relates to a novel use of biodegradable/biocompatible, non-toxic starch based mucoadhesive copolymeric carrier systems grafted with polyacrylic acid, and optionally thiolated, for manufacturing of vaginal drug delivery systems. The invention also pertains to novel use of carrier systems comprising starch based mucoadhesive copolymers grafted with polyacrylic acid and also thiolated, as well as to a method for their production. In particularly preferred embodiments, the invention is realized in the veterinary field by way of a novel use for administering progesterone via vaginal route of a sheep in order to ensure estrus synchronization.

### Background of the Invention

It is known in the prior art that, in the preparations used in parenteral and oral therapies, there are a number of disadvantages encountered in terms of efficiency, frequency of application and necessity of repetitive applications. Especially, orally administrated drugs undergo the first past effect in the liver and their bioavailability is reduced and the side effects increase. However, in the case of injectable preparations, there is an irritation and/or abscess formation besides pain in the patient. The controlled releases of drug in the sustained periods can be obtained by the result of intensive contact due to the mucoadhesive carrier in the absorption center which is namely mouth, nose, gastrointestinal system and inside of vagina. Accordingly, the bioavailability and the patient compliance of a drug can be increased and the release rate can be changed (Levy et al., 2000, Steroids, 65: 645). Polymers which perform controlled drug release and used via vaginal route can also be used for antibiotic and antimycotic therapies as well as for purposes of hormone supply, especially progesterone and testosterone (Schnürch et al., 2001, Journal of Controlled Release, 71: 277; and Kast and Schnürch, 2001, Biomaterials, 22: 2345).

"Bioadhesion" is generally defined as attachment of natural or synthetic polymers into biological structures/substrates. It is occurred from any bond formation between two biological surfaces or between a biological and a synthetic surface. If this bond is between mucus and polymer which is the carrier ingredient of bioadhesive material (matrix), in this case adhesion to mucosa (mucoadhesion) can be mentioned. Accordingly, if the attached structure, namely substrate, is a biological substrate wherein the mucosal membranes exist such as eye, mouth, nasal cavity, gastrointestinal system and vagina, this property is called as "mucoadhesiveness /mucoadhesion" for describing a more specific situation as bioadhesive systems are applied into mucosal membranes. In other words, mucoadhesion is defined as an ability of synthetic or natural macromolecules to adhere to the mucosal tissues such as mucosa of small intestine. Similarly, in the bioadhesive drug delivery systems, the term of "bioadhesion" is used to define the adhesion between synthetic and natural polymers and soft tissues (e.g. gastrointestinal mucosa).

The mucoadhesive property can be brought to various types of natural and synthetic polymers and, if any, these properties of polymers can be improved. It is accepted that the mucoadhesion properties of polymers is essentially derived from the ionic interactions with mucus glycoproteins, interactions based on non-covalent bonding such as hydrogen bonds and Van der Waal's forces. Following these interactions, the diffusion of polymer chains into mucus layers occur by the formation of electrostatic, hydrophobic or hydrogen bonding at the interface in between mucin and polymer. The sustained release systems prepared by using natural polymers can be applied by vaginal, rectal, oral, nasal, ocular, pulmonary, buccal and topical administration routes. Vaginal route *inter alia* would be particularly suitable for administering bioadhesive drug carrier systems by virtue of the mucosal media. The systems to be applied via these administration routes may be solution, gel, suspensions with nanoparticular and microparticular systems, ovule, pessary, vaginal suppository.

Vaginal route has a number of advantages compared to other routes of applications. The major advantages are that the vagina has a large surface area and the vascularization is too much and also that vagina has a low enzymatic activity and its epithelial tissue has a high absorption potential. The vaginal route is preferred for a number of drugs (hormones, antimycotics, peptides and proteins etc.) for the local or systemic effect. The preparation applied reaches uterus by vaginal absorption (uterine first past effect) and reaches higher tissue concentrations in there as compared to the application by oral or injection routes (Ameye et al., 2002, Journal of Controlled Release, 79: 173).

As already known, it is an important issue in sheep breeding to obtain breeding three times in two years by estrus synchronization and in this way to ensure economic gains to the breeder. With the recently applied estrus synchronization techniques, while both progesterone and prostaglandin injections can be applied in the breeding season, only progesterone injection can be used out of the season. The problem encountered here is the necessity of application that the injection must be applied repeatedly. This causes the labor intensity and reduces its applicability. There are subcutaneous implants which can be applied apart from the injection method. However, it is necessary to apply these implants under the skin by a small surgery operation and then to remove them after the application period. This procedure did not gain an importance as it is again a technique in which the complications are frequently encountered as well as it is troublesome and time consuming.

Nowadays, the controlled drug release apparatus containing natural or synthetic progesterone (sponge, CIDR etc.) which are placed into vagina to control of reproductive activities in terms of the animal health and especially for the purpose of estrus synchronization in the area of reproductive biotechnology of farm animals, are often used. Among them, the apparatus which are commonly used in sheep for estrus synchronization by placing into vagina are the sponges which are sold in various trade names such as of Senkron Sponge or Syncro-Part impregnated progestagens i.e. 60 mg medroxyprogesterone acetate which is a synthetic progesterone or fluorogestone acetate (30-40 mg for sheep and 45 mg for goats).

Vaginal sponges are generally hold for 14 days in vagina following application. During this period, even if it is rare, some complications related to irritation, sponge displacement can be encountered and in the case if the hymen ring is too visible and if the tract is narrow, the difficulties during the vaginal sponge application can be encountered. Similar problems and complications are available in the process of sponge removal from vagina after a certain time. Another device which is used for the similar purpose and which can be applied into vagina is CIDR containing 1.38 g or 1.9 g of progesterone for cows and 300 mg progesterone for sheep. The similar application and removal procedures into vagina such as sponge applications are also carried out when this device is used. Accordingly, the similar problems are encountered which may appear during the vaginal sponge application. In this case, the requirement for designing of the alternative mucoadhesive and non-toxic drug delivery systems which can eliminate the disadvantages of intravaginal systems, which have high efficiency and provide convenience in the application (e.g. after the application, without needing to be withdrawn) arises. This situation will provide economical contribution to the livestock breeding along with its easy applicability.

The natural progesterone does not have any androgenic activity and therefore it can be commonly used for longer periods in order to help the control of the reproduction. But, the orally administrated synthetic progesterone is commonly used for several gynecological problems such as endometrial hyperplasia, uterus bleedings, uterus functional disorders, endometriosis, premenstrual syndrome and luteal phase disorders in human health. The supplement of progesterone is necessary both in a part of endometrial preparation in the oocyte donation programs and in GnRH analogue regulations in the in vitro fertilization cycle. The biggest problem here is the way of drug application. Although the most proper way is oral administration, the gastrointestinal metabolism prevents the adequate absorption of progesterone. Besides this, the drug undergoes the first past effect in the liver. Intramuscular administration may not always be preferred because it causes pain and irritation, and abscess formation. For these reasons, the controlled vaginal administrations are preferred in the applications (Trimnell et al., 2003, Journal of Applied Polymer Science, 17 (5): 1607).

### Brief description of the Invention

The present invention aims at providing a novel use of biodegradable/biocompatible, non-toxic starch based mucoadhesive copolymeric carrier systems grafted with polyacrylic acid, and optionally thiolated, and by way of these systems, development of suitable pharmaceutical formulations preferably in tablet form for administering of a pharmaceutically active ingredient to animals by means of a trans-mucosal administration by vaginal route (e.g. administering progesterone to sheep by vaginal route) in a controlled or extended release regime. This invention, as a model in the veterinary medicine field, also comprises the applications of the progesterone release via vaginal route for the purpose of estrus synchronization in the sheep. The invention also provides production of the modified starch based mucoadhesive biocompatible pharmaceutical forms, preferably vaginal tablets that can administer the progesterone hormone via vaginal route in a controlled manner for the purpose of estrus synchronization in the sheep. For this purpose, in the presented invention, the starch based copolymers which have biodegradable/ biocompatible, non-toxic and mucoadhesive properties were synthesized and characterized as an active drug carrier system. The usability of these copolymers in vaginal route, and particularly use of these polymers in the area of veterinary medicine was first time examined in the formulations of drug carrier system in sheep via vaginal route within scope of the present invention.

Starch-graft-poly(acrylic acid) copolymers (S-g-PAA) were synthesized by the chemically initiated graft copolymerization method using four different types of starch [maize (MS), wheat (WS), potato (PS) and rice (RS)] and acrylic acid monomer (AA). S-g-PAA products are thiolated using different thiolation reagents first time in this invention. All synthesized copolymers (grafted and thiolated) were characterized and their cytotoxicity tests were performed. The bioadhesive tablets containing hormone were prepared by the selected copolymers. With these tablets, the *in vitro* tests were performed. The tablets containing the progesterone hormone which were prepared using the synthesized copolymers were administrated to sheep via vaginal route. The *in vivo* hormone release profiles obtained were compared with the two commercial products used in the market for sheep (vaginal sponge and CIDR).

### ABBREVIATIONS AND DEFINITIONS CONCERNING POLYMER PRODUCTS

RS: As used herein, the abbreviation "RS" refers to starches which were obtained from rice.
WS: As used herein, the abbreviation "WS" refers to starches which were obtained from wheat.
PS: As used herein, the abbreviation "PS" refers to starches which was obtained from potato.
MS: As used herein, the abbreviation "MS" refers to starches which was obtained from maize.
S-g-PAA: As used herein, the abbreviation "S-g-PAA" refers to starch-graft-poly(acrylic acid) copolymers which were obtained using different type of starches.
(S-g-PAA)_{ng}: As used herein, the abbreviation "(S-g-PAA)_{ng}" refers to starch-graft-poly(acrylic acid) copolymers which gelatinization process did not apply before the grafting reaction. The abbreviation also covers the products which were called as non-gelatinized starch-graft-poly(acrylic acid) copolymers.
(S-g-PAA)_{ngc}: As used herein, the abbreviation "(S-g-PAA)_{ngc}" refers to starch-graft-poly(acrylic acid) copolymers which gelatinization process did not apply before the grafting reaction and a crosslinker was used in the presence of the grafting reaction. The abbreviation also covers the products which were called as non-gelatinized and crosslinked starch-graft-poly(acrylic acid) copolymers.
(S-g-PAA)_{g}: As used herein, the abbreviation "(S-g-PAA)_{g}" refers to starch-graft-poly(acrylic acid) copolymers which gelatinization process were applied before the grafting reaction. The abbreviation also covers the products which were called as gelatinized starch-graft-poly(acrylic acid) copolymers.
(S-g-PAA)_{gc}: As used herein, the abbreviation "(S-g-PAA)_{gc}" refers to starch-graft-poly(acrylic acid) copolymers which gelatinization process were apply before the grafting reaction and a crosslinker was used in the presence of the grafting reaction. The abbreviation also covers the products which were called as gelatinized and crosslinked starch-graft-poly(acrylic acid) copolymers.
(RS-g-PAA)_{ng}: As used herein, the abbreviation "(RS-g-PAA)_{ng}" refers to rice starch-graft-poly(acrylic acid) copolymers which gelatinization process did not apply before the grafting reaction. The abbreviation also covers the products which were called as non-gelatinized rice starch-graft-poly(acrylic acid) copolymers.
(RS-g-PAA)_{ngc}: As used herein, the abbreviation "(RS-g-PAA)_{ngc}" refers to rice starch-graft-poly(acrylic acid) copolymers which gelatinization process did not apply before the grafting reaction and a crosslinker was used in the presence of the grafting reaction. The abbreviation also covers the products which were called as non-gelatinized and crosslinked rice starch-graft-poly(acrylic acid) copolymers.
(RS-g-PAA)_{g}: As used herein, the abbreviation "(RS-g-PAA)_{g}" refers to rice starch-graft-poly(acrylic acid) copolymers which gelatinization process were applied before the grafting reaction. The abbreviation also covers the products which were called as gelatinized rice starch-graft-poly(acrylic acid) copolymers.
(RS-g-PAA)_{gc}: As used herein, the abbreviation "(RS-g-PAA)_{gc}" refers to rice starch-graft-poly(acrylic acid) copolymers which gelatinization process were apply before the grafting reaction and a crosslinker was used in the presence of the grafting reaction. The abbreviation also covers the products which were called as gelatinized and crosslinked rice starch-graft-poly(acrylic acid) copolymers.
(WS-g-PAA)_{ng}: As used herein, the abbreviation "(WS-g-PAA)_{ng}" refers to wheat starch-graft-poly(acrylic acid) copolymers which gelatinization process did not apply before the grafting reaction. The abbreviation also covers the products which were called as non-gelatinized wheat starch-graft-poly(acrylic acid) copolymers.
(WS-g-PAA)_{ngc}: As used herein, the abbreviation "(WS-g-PAA)_{ngc}" refers to wheat starch-graft-poly(acrylic acid) copolymers which gelatinization process did not apply before the grafting reaction and a crosslinker was used in the presence of the grafting reaction. The abbreviation also covers the products which were called as non-gelatinized and crosslinked wheat starch-graft-poly(acrylic acid) copolymers.
(WS-g-PAA)_{g}: As used herein, the abbreviation "(WS-g-PAA)_{g}" refers to wheat starch-graft-poly(acrylic acid) copolymers which gelatinization process were applied before the grafting reaction. The abbreviation also covers the products which were called as gelatinized wheat starch-graft-poly(acrylic acid) copolymers.
(WS-g-PAA)_{gc}: As used herein, the abbreviation "(WS-g-PAA)_{gc}" refers to wheat starch-graft-poly(acrylic acid) copolymers which gelatinization process were apply before the grafting reaction and a crosslinker was used in the presence of the grafting reaction. The abbreviation also covers the products which were called as gelatinized and crosslinked wheat starch-graft-poly(acrylic acid) copolymers.
(PS-g-PAA)_{ng}: As used herein, the abbreviation "(PS-g-PAA)_{ng}" refers to potato starch-graft-poly(acrylic acid) copolymers which gelatinization process did not apply before the grafting reaction. The abbreviation also covers the products which were called as non-gelatinized potato starch-graft-poly(acrylic acid) copolymers.
(PS-g-PAA)_{ngc}: As used herein, the abbreviation "(PS-g-PAA)_{ngc}" refers to potato starch-graft-poly(acrylic acid) copolymers which gelatinization process did not apply before the grafting reaction and a crosslinker was used in the presence of the grafting reaction. The abbreviation also covers the products which were called as non-gelatinized and crosslinked potato starch-graft-poly(acrylic acid) copolymers.
(PS-g-PAA)_{g}: As used herein, the abbreviation "(PS-g-PAA)_{g}" refers to potato starch-graft-poly(acrylic acid) copolymers which gelatinization process were applied before the grafting reaction. The abbreviation also covers the products which were called as gelatinized potato starch-graft-poly(acrylic acid) copolymers.
(PS-g-PAA)_{gc}: As used herein, the abbreviation "(PS-g-PAA)_{gc}" refers to potato starch-graft-poly(acrylic acid) copolymers which gelatinization process were apply before the grafting reaction and a crosslinker was used in the presence of the grafting reaction. The abbreviation also covers the products which were called as gelatinized and crosslinked potato starch-graft-poly(acrylic acid) copolymers.
(MS-g-PAA)_{ng}: As used herein, the abbreviation "(MS-g-PAA)_{ng}" refers to maize starch-graft-poly(acrylic acid) copolymers which gelatinization process did not apply before the grafting reaction. The abbreviation also covers the products which were called as non-gelatinized maize starch-graft-poly(acrylic acid) copolymers.
(MS-g-PAA)_{ngc}: As used herein, the abbreviation "(MS-g-PAA)_{ngc}" refers to maize starch-graft-poly(acrylic acid) copolymers which gelatinization process did not apply before the grafting reaction and a crosslinker was used in the presence of the grafting reaction. The abbreviation also covers the products which were called as non-gelatinized and crosslinked maize starch-graft-poly(acrylic acid) copolymers.
(MS-g-PAA)_{g}: As used herein, the abbreviation "(MS-g-PAA)_{g}" refers to maize starch-graft-poly(acrylic acid) copolymers which gelatinization process were applied before the grafting reaction. The abbreviation also covers the products which were called as gelatinized maize starch-graft-poly(acrylic acid) copolymers.
(MS-g-PAA)_{gc}: As used herein, the abbreviation "(MS-g-PAA)_{gc}" refers to maize starch-graft-poly(acrylic acid) copolymers which gelatinization process were apply before the grafting reaction and a crosslinker was used in the presence of the grafting reaction. The abbreviation also covers the products which were called as gelatinized and crosslinked maize starch-graft-poly(acrylic acid) copolymers.
RS-Cys: As used herein, the abbreviation "RS-Cys" refers to thiolated rice starch which were synthesized by using L-Cysteine hydrochloride monohydrate (Cys) as thiolation reagent.
RS-TGA: As used herein, the abbreviation "RS-TGA" refers to thiolated rice starch which were synthesized by using thioglycolic acid (TGA) as thiolation reagent.
RS-ITH: As used herein, the abbreviation "RS-ITH" refers to thiolated rice starch which were synthesized by using 2-Iminothiolane hydrochloride (2-ITH) as thiolation reagent. PAA-Cys: As used herein, the abbreviation "PAA-Cys" refers to thiolated poly(acrylic acid) which were synthesized by using L-Cysteine hydrochloride monohydrate (Cys) as thiolation reagent.
PAA-TGA: As used herein, the abbreviation "PAA-TGA" refers to thiolated poly(acrylic acid) which were synthesized by using thioglycolic acid (TGA) as thiolation reagent. PAA-ITH: As used herein, the abbreviation "PAA-ITH" refers to thiolated poly(acrylic acid) which were synthesized by using 2-Iminothiolane hydrochloride (2-ITH) as thiolation reagent.
(RS-g-PAA)_{gc}-Cys: As used herein, the abbreviation "(RS-g-PAA)_{gc}-Cys" refers to thiolated gelatinized and crosslinked rice starch-graft-poly(acrylic acid) copolymers (RS-g-PAA)_{gc} which were synthesized by using L-Cysteine hydrochloride monohydrate (Cys) as thiolation reagent.
(RS-g-PAA)_{gc}-TGA: As used herein, the abbreviation "(RS-g-PAA)_{gc}-TGA" refers to thiolated gelatinized and crosslinked rice starch-graft-poly(acrylic acid) copolymers (RS-g-PAA)_{gc} which were synthesized by using thioglycolic acid (TGA) as thiolation reagent.
(RS-g-PAA)_{gc}-ITH: As used herein, the abbreviation "(RS-g-PAA)_{gc}- ITH" refers to thiolated gelatinized and crosslinked rice starch-graft-poly(acrylic acid) copolymers (RS-g-PAA)_{gc} which were synthesized by using 2-Iminothiolane hydrochloride (2-ITH) as thiolation reagent.
(RS-g-PAA)_{ngc}-TGA: As used herein, the abbreviation "(RS-g-PAA)_{ngc}-TGA" refers to thiolated non-gelatinized and crosslinked rice starch-graft-poly(acrylic acid) copolymers (RS-g-PAA)_{ngc} which were synthesized by using thioglycolic acid (TGA) as thiolation reagent.
(WS-g-PAA)_{ngc}-Cys: As used herein, the abbreviation "(WS-g-PAA)_{ngc}-Cys" refers to thiolated non-gelatinized and crosslinked wheat starch-graft-poly(acrylic acid) copolymers (WS-g-PAA)_{ngc} which were synthesized by using L-Cysteine hydrochloride monohydrate (Cys) as thiolation reagent.
(WS-g-PAA)_{ngc}-TGA: As used herein, the abbreviation "(WS-g-PAA)_{ngc}-TGA" refers to thiolated non-gelatinized and crosslinked wheat starch-graft-poly(acrylic acid) copolymers (WS-g-PAA)_{ngc} which were synthesized by using thioglycolic acid (TGA) as thiolation reagent.
(WS-g-PAA)_{gc}-Cys: As used herein, the abbreviation "(WS-g-PAA)_{gc}-Cys" refers to thiolated gelatinized and crosslinked wheat starch-graft-poly(acrylic acid) copolymers (WS-g-PAA)_{gc} which were synthesized by using L-Cysteine hydrochloride monohydrate (Cys) as thiolation reagent.
(WS-g-PAA)_{gc}-TGA: As used herein, the abbreviation "(WS-g-PAA)_{gc}-TGA" refers to thiolated gelatinized and crosslinked wheat starch-graft-poly(acrylic acid) copolymers (WS-g-PAA)_{gc} which were synthesized by using thioglycolic acid (TGA) as thiolation reagent.
(WS-g-PAA)_{gc}-ITH: As used herein, the abbreviation "(WS-g-PAA)_{gc}-ITH" refers to thiolated gelatinized and crosslinked wheat starch-graft-poly(acrylic acid) copolymers (WS-g-PAA)_{gc} which were synthesized by using 2-Iminothiolane hydrochloride (2-ITH) as thiolation reagent.
AA: acrylic acid
CAN: Ammonium cerium (IV) nitrate
NMBA: *N,N'*-Methylenebisacrylamide
EDAC: *N-*(3-Dimethylaminopropyl)-*N'*-ethylcarbodiimide hydrochloride
Cys: L-Cysteine hydrochloride monohydrate
TGA: Thioglycolic acid
2-ITH: 2-Iminothiolane hydrochloride
GA: Graft amount
ME: Matrix erosion
Qₑ: Equilibrium swelling degree

### Brief Description of the Figures

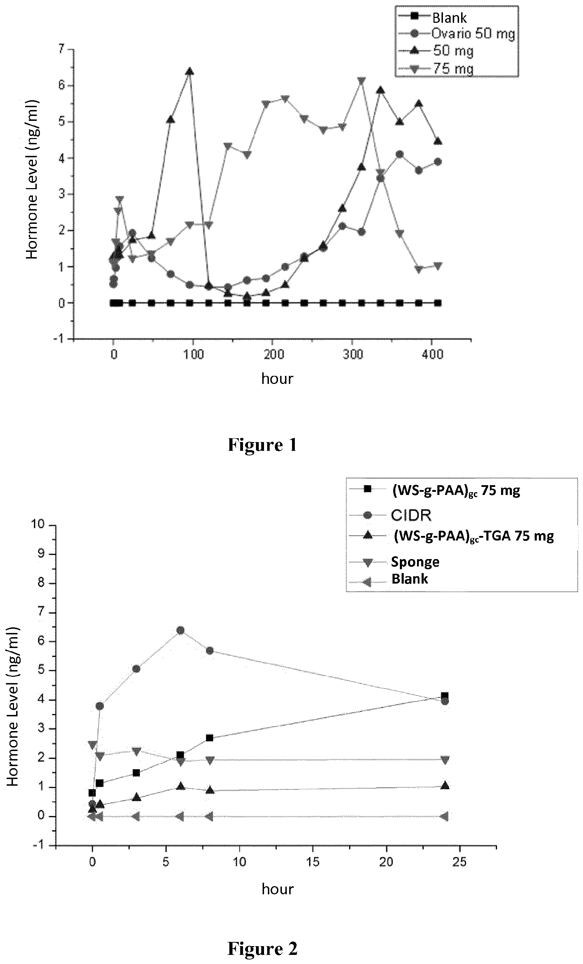

Fig. 1 shows progesterone hormone levels (ng/ml) in blood serum of the sheep belonging to the groups of sheep with no vaginal treatment (blank), sheep treated with (WS-g-PAA)_{gc} carrier tablets containing 50 mg and 75 mg progesterone hormone, and ovariectomized sheep treated with (WS-g-PAA)_{gc} carrier tablets containing 50 mg progesterone hormone.
Fig. 2 shows progesterone hormone levels (ng/ml) in the blood serum of sheep which are vaginally non-treated (blank) sheep, sponge applied sheep, CIDR applied sheep, sheep treated with (WS-g-PAA)_{gc} carrier tablets containing 75 mg progesterone hormone, and sheep treated with (WS-g-PAA)_{gc}-TGA carrier tablets containing 75 mg progesterone hormone, wherein the progesterone hormone levels (ng/mL) in blood serum of the sheep are shown for the first 24 hours.

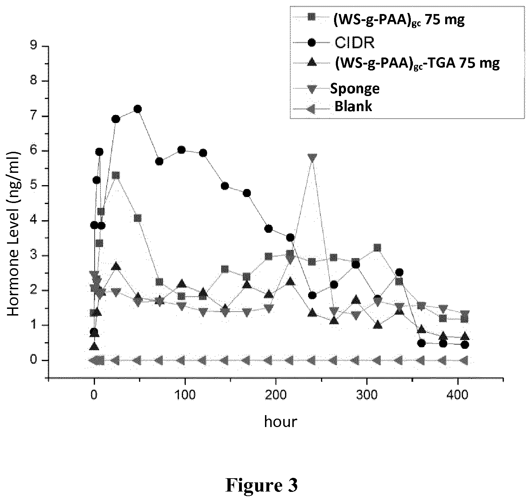

Fig. 3 shows progesterone hormone levels (ng/ml) in the blood serum of sheep which are vaginally non-treated (blank) sheep, sponge applied sheep, CIDR applied sheep, sheep treated with (WS-g-PAA)_{gc} carrier tablets containing 75 mg progesterone hormone, and sheep treated with (WS-g-PAA)_{gc}-TGA carrier tablets containing 75 mg progesterone hormone.

### Detailed Description of the Invention

The purpose of this invention is realizing an advantageous use of biodegradable/biocompatible, non-toxic mucoadhesive starch based copolymers which are polyacrylic acid grafted, and optionally thiolated on graft chain of the starch molecule, in drug carrier systems directed to applications in mucosal environments, and development of suitable pharmaceutical forms preferably in tablet form for administering of a pharmaceutically active ingredient to animals (e.g. administering progesterone to sheep by vaginal route) in a controlled release regime. It is disclosed also, as an exemplary embodiment in the veterinary field, an application model for administering progesterone via vaginal route of sheep in order to ensure estrus synchronization.

Accordingly, one object of the present invention is to provide a novel use of polyacrylic acid grafted starch based copolymers, or thiolated starch-graft-polyacrylic acid copolymers which are thiolated on graft chain of the starch molecule, in manufacturing of extended release drug carrier systems directed to trans-mucosal applications.

Another object of the present invention is to provide novel use of polyacrylic acid grafted starch based copolymers which are thiolated according to new methods as provided hereinbelow.

A more specific object of the present invention is use of the copolymers described herein within mucoadhesive and non-toxic drug carrier systems, preferably in tablet formulations, which can eliminate the major disadvantages of intravaginal systems (for example sponge and CIDR etc.) with estrus synchronization purpose in veterinary medicine and may be an alternative to these systems, and which afford the convenience in the application; for example of the type which can be placed and never removed, and which can be applied at once.

A further object of the present invention is to provide use of starch based graft copolymers with biodegradable/biocompatible, non-toxic and mucoadhesive properties for applying progesterone hormone via vaginal route in the sheep for the aim of estrus synchronization and to provide its controlled release.

A further object of the present invention is to provide a new use of these synthesized copolymers in the area of veterinary medicine in the drug carrier system formulations in the form of tablets for use via vaginal route in sheep.

This invention encompasses use of the copolymers described herein in a drug carrier system to apply, for instance a pharmaceutically active compound and/or hormone(s), and comprises mainly biodegradable, biocompatible, hydrogel and mucoadhesive polysaccharide derivative copolymers.

One of the properties of the carrier system mentioned herewith is that the polysaccharide derivative copolymer is preferably a starch based copolymer.

Another property of the carrier system mentioned in this invention is that the starch based copolymer is a starch-graft-poly(acrylic acid) copolymer (S-g-PAA).

The starch-graft-poly(acrylic acid) copolymer mentioned in this invention is synthesized from a starch which is selected preferably from wheat, rice, potato and maize starches.

The starch-graft-poly(acrylic acid) copolymer mentioned in this invention is synthesized from a starch preferably selected from wheat starch.

This invention encompasses also the use of a drug carrier system to apply a pharmaceutically active compound and/or hormone(s) into an animal body, wherein the carrier comprises mainly biodegradable, biocompatible, hydrogel and mucoadhesive starch-graft-poly(acrylic acid) copolymer polysaccharide derivative copolymers, preferably thiolated copolymers.

According to another aspect of the present invention, there is provided a new use of a drug carrier system to apply a pharmaceutically active compound and/or hormone(s) into an animal body, wherein the carrier comprising mainly biodegradable, biocompatible, hydrogel and mucoadhesive starch-graft-poly(acrylic acid) copolymer polysaccharide derivative copolymers, which copolymers can be selected from thiolated and/or non-thiolated forms.

In preferred embodiments, the invention encompasses a new use of a drug carrier system to apply the drug active compound and/or hormone(s) into an animal body, and polysaccharide derivative starch based thiolated copolymer with biodegradable, biocompatible, hydrogel and mucoadhesive properties is obtained by a thiolation reaction using starch and L-cysteine hydrochloride monohydrate (Cys) or thioglycolic acid (TGA) or 2-iminothiolane hydrochloride (2-ITH) compounds.

The drug carrier systems prepared with the synthesized new copolymers mentioned in this invention can be administrated via vaginal administration route.

Said drug carrier systems may comprise drugs with the local or systemic effect preferably for the administration via vaginal route.

In a preferred embodiment, the drug carrier system mentioned in this invention comprises a hormone which provides estrus synchronization.

The hormone mentioned in this invention is preferably progesterone.

The drug carrier system according to the present invention can be combined with a hormone which provides estrus synchronization.

The hormone according to this invention is preferably progesterone.

Dosage of progesterone mentioned in this invention is 50-300 mg, preferably 50-75 mg and more preferably 75 mg.

The dosage form of vaginal drug delivery systems is selected from the group including tablet, pessary, ovule, vaginal suppository and gel forms.

The dosage form of vaginal drug delivery systems is preferably in tablet form.

The tablet dosage forms according to this invention are preferably in the weight of 300 mg-1.75 g and in a diameter of 1.5 cm, and preferably round in shape.

The work of adhesion (mJ/cm²) value of starch-graft-poly(acrylic acid) copolymer and thiolated product as provided herein is preferably in a range of 0.05-7.00 and their maximal detachment force (N/cm²) is preferably in a range of 0.05 -3.00.

The equilibrium swelling degree (Qₑ) (g H₂O/g product) of starch-graft-poly(acrylic acid) copolymers and thiolated products mentioned in this invention is preferably in the range of 5.00 - 110.00 and their matrix erosion (ME%) is preferably in the range of 10-60%.

The graft amount (GA%) of starch-graft-poly(acrylic acid) copolymers mentioned in this invention is in the range of between 21.32 ± 0.5% and 24.84 ± 2.0%.

The thiol group amount of thiolated products of starch-graft-poly(acrylic acid) copolymers within the present invention is in the range of 8-305 µmole/g polymer.

Following vaginal administration to sheep, the copolymer based carrier systems according to this invention produce a controlled release such that the serum progesterone level is ≥ 1 ng/ml at 1 st day and above 1 ng/ml for at least during 12-14 days.

### Formulations of Modified Starch Based Carrier Systems for In Vivo Vaginal Drug Delivery

The applied procedures to prepare modified starch based vaginal carrier systems, preferably in tablet form, consist of four main categories described below:
*1. The synthesis of starch based graft copolymers (with acrylic acid monomer) and other modified polymers (thiomers):*
   - Determination of the effective parameters in the reactions,
   - Optimization of the reaction conditions according to these parameters,
   - Synthesis, purification and characterization with Fourier Transform Infrared Spectrophotometer (FTIR) technique and determination of the graft amount (wt.%) (GA%) of polymers in the reaction conditions determined following the optimization.
*2. Cytotoxicity studies:*
   - Performing cytotoxicity studies of the synthesized starch based graft copolymers.
*3. Determination of product properties for the use of these products in vaginal tablet formulations:*
   - The preparation of tablets using all of the synthesized and characterized copolymers,
   - The in vitro determination of swelling, matrix erosion and mucoadhesion properties into vaginal mucosa of sheep.
*4. Performing in vivo experiments*
   - The preparation of tablets comprising synthesized starch based copolymer and inert dye for investigating the behaviour in sheep vagina,
   - The preparation of tablets comprising progesterone hormone by using product(s) which is/are successful according to the results obtained with the application of dye containing tablets,
   - The *in vivo* investigation of progesterone release properties via sheep vagina route using progesterone containing tablets out of the breeding season,
   - According to the results obtained; the *in vivo* investigation of progesterone release properties via sheep vagina route using different amount of progesterone containing tablets of different weight out of the breeding season,
   - Carrying out the progesterone analysis in the serum separated from collected blood samples via RIA method, evaluating all results and optimizing working conditions according to the most proper result.

### Synthesis of S-g-PAA Copolymers

**Reaction system:** The production of S-g-PAA polymers were carried out in a 5 necked glass reaction system (mechanical stirrer, condenser, thermometer, gas passing and feeding systems) inside a water bath placed onto a magnetic stirrer with a contact thermometer which can control the heat with ±1°C sensitivity.

**Experimental Procedure:** Reactions were carried out by using MS, WS, PS and RS as follows:
- Before carrying out the grafting reaction: By applying the gelatinization pretreatment at the specific gelatinization temperature of each starch species (S-g-PAA)_{g} and without applying this pretreatment (S-g-PAA)_{ng},
- Grafting reactions were carried out by using crosslinker (S-g-PAA)_{ngc} and without using crosslinker (S-g-PAA)_{ng} according to the chemically initiated free radical addition polymerization mechanism which is known as a common method.

The starches (MS, WS, PS and RS) and the initiator were dried in an oven at 110°C before using in the reaction. In the case gelatinization pretreatment is applied, the starch samples were gelatinized by mixing until they become opaque at gelatinization temperatures in water (4 g starch/108 ml distilled water) and then they cooled. Then, the grafting reaction was continued as indicated below.

When gelatinization pretreatment was not applied, the grafting reaction was carried out with the same starch types in aqueous medium (4 g starch/ 183 ml distilled water) according to the free radical addition polymerization mechanism and by using a chemical initiator CAN (0.005 mol/L) and AA monomer in an acidic media (0.5 mol/L HNO₃) for 3 hours. In the reactions where the crosslinker was used, NMBA amount was used as 0.05% of the AA monomer by weight. All graft reactions were performed under the stream of nitrogen gas at 35±1°C with the step by step addition of substances into the reactor. The purification of the product was carried out as described below.

Accordingly, the reactions were carried out in four experimental group for each starch types namely the gelatinized crosslinked, the gelatinized without crosslinked, the non-gelatinized crosslinked and the non-gelatinized without cross-linked.

**Purification of Graft Copolymer:** The purification procedure of the obtained S-g-PAA copolymers by separating from the reaction media was performed differently in the cases where the gelatinization was performed and not performed.

In the case where the gelatinization was not performed, the solid and liquid phase produced at the end of reaction were separated by filtration. Then, it was repeatedly washed with warm water by applying the filtration to remove homopolymer which may form, even a little, during the reaction from the copolymer product. After completing the washing procedure, the copolymer product was dried in a vacuum oven at 50 °C.

In the case of the gelatinization is performed, the reaction mixture was heated to 60°C and neutralized with 10% sodium hydroxide solution to pH 10. Following this, it was precipitated by mixing in cold methanol, filtered, washed with ethyl alcohol and dried in a vacuum oven at 50°C.

### Synthesis of Thiolated Starch Copolymers (Thiomers)

**Reaction System:** The reaction system in which the above mentioned graft reactions was performed was used in the thiolation reactions.

**Experimental Procedure:** To obtain thiolated starch copolymer products, first of all, the optimization experiments of reaction conditions were done. Cys was primarily selected as thiolation reagent and the work was carried out according to the following schedule:
- Thiolation of starch with Cys
- Thiolation of S-g-PAA copolymers
- Thiolation of PAA for comparison purpose
- Synthesis with different thiolation reagents (TGA and 2-ITH)

All reactions were performed at room temperature and under a stream of nitrogen gas. EDAC is used to increase the activation of compounds to be thiolated. To protect sulphydryl groups from the oxidation, the reaction was carried out at room temperature at pH 4 and 5. In thiolation reactions, PAA, starch and starch compounds were mixed in water in a reactor flask (1g compound/250 mL distilled water). Then EDAC (50 mM) and Cys (4g) were added and the mixture was kept at room temperature for 3 hours. In case TGA and 2 ITH were used, having the same experimental conditions, 1% aqueous solution of the compound to be thiolated and the amount of the thiolation reagent was the same as the compound to be thiolated.

**The Purification of Thiomers:** To purify the thiomer products, the reaction mixture obtained at the end of the thiolation reaction were dialyzed with 5mM hydrochloric acid at 10±1°C (dialyze tubes Medicell International Ltd, Size 3 Inf Die 20/32-15,9 mm, MWCO-12-14000 Daltons) once, with 5mM HCl containing 1% sodium chloride twice and with 1mM HCl containing 1% sodium chloride twice. Following the adjustment of pH of the thiolated polymer conjugates, which were dialyzed, to pH 5, they were lyophilized at 0.01 mbar pressure at -30±1°C (Lyovac GT2E, Steris, Germany) and the products were kept at +4°C.

### Characterization of S-g-PAA Copolymers

- In the study, all the S-g-PAA products which were synthesized by using four different types of starch were obtained as a white or creamy coloured solid substance.
- The structure of the starch based copolymers as synthesized were identified by Fourier Transform Infrared Spectrophotometer (FTIR) technique. The analysis was performed using Digilab Excalibur-FTS 3000 MX model FT-IR apparatus. In FTIR analysis, the tablets prepared by dilution in a sample/KBr ratio of 1/200 were used and recorded at a wavenumber range of 400-4000 cm⁻¹.
- The FTIR spectra of MS, PS, WS and RS were same and the characteristic absorption peaks of spectra were as follows: the deep and broad peak belonging to glucosidic ring in the structure of starch, at 3200-3450 cm⁻¹ region, the peak belonging to the C-H bond stretching vibration at 2927-2931 cm⁻¹ region (maximum at 2930 cm⁻¹), the deep and broad peak belonging to the stretching vibration of free methylol groups at 1450-1250 cm⁻¹ region and the broad peak belonging to the stretching vibration of C-O bond because of the free OH group in the structure at 1157-1080 cm⁻¹ region.

- The FTIR spectra of S-g-PAA graft products show similarity because the graft reaction took place in all of the products. In the FTIR spectra of all products, it was seen that the grafting reaction of poly(acrylic acid) (as sodium salt) to starch occurred. That is, while a significant reduction in the peak intensity of the broad and deep peak because of the stretching vibration of free methylol groups at 1470-1250 cm⁻¹ region, as the products were neutralized with NaOH solution in the purification of graft copolymers, the new absorption peaks were observed at a maximum of 1560 and 1410 cm⁻¹ belonging to asymmetric and symmetric stretching vibrations of R-COONa (carboxylate) due to the conversion of grafted poly(acrylic acid) group into the sodium salt.
- The graft amount wt% (GA%) of grafted copolymers was determined by a volumetric method (Athawale and Lele, 1998, Carbohydrate Polymers, 35: 21) . The GA% values of synthesized copolymers varied between 21.32±0.5% and 24.84±2.0%.
- The synthesized starch graft copolymers were weighted in an amount of 70 mg and the tablets were prepared at 5000 psi pressure and the equilibrium swelling degree (Qₑ) (gH₂O/g product) (Dalaran et al., 2011, Desalination, 279: 170) and the matrix erosion (wt%) (ME%) values were determined in pH 5 lactate buffer for the investigation of their behaviour in vagina *in vitro* (Cevher et al., 2008, AAPS Pharm Sci. Tech., 9(3): 953). It was observed that the tablets prepared from (PS-g-PAA)_{gc}, (WS-g-PAA)_{g}, (WS-g-PAA)_{gc} and (RS-g-PAA)_{gc} products were remained without disintegration in the media. Qₑ (g H₂O/g product) and ME% values in pH 5 lactate buffer at the end of 24 hours were determined to be 19.60 ±1.19 and 43.60±8,70% for (PS-g-PAA)_{gc} product; 12.21±1.23 and 57.80±6.83% for (WS-g-PAA)g product; 17.65±1.19 and 24.60±2.54 % for (WS-g-PAA)_{gc} product, 17.68±1.66 and 17.83±3.41% for (RS-g-PAA)_{gc} respectively.
- The mucoadhesion properties of the products, the work of adhesion and maximal detachment force of tablets were determined by using TA-XTPlus Texture Analyser (Stable Microsystems, Haslemere, UK) apparatus with 5 kg loading capacity (Cevher et al.,2008, AAPS Pharm Sci. Tech., 9(3): 953). The work of adhesion (mJ/cm²) and the maximal detachment force of tablets (N/cm²) were calculated by using Texture Exponent 4.0.4.0 software programme. The work of adhesion (mJ/cm²) and the maximal detachment force (N/cm²) of tablets prepared from S-g-PAA product were determined to be 0.626±0.035 and 0.675±0.016 for (PS-g-PAA)_{gc}, 0.599±0.019 and 0.546±0.210 for (WS-g-PAA)g product, 0.709±0.024 and 0.644±0.016 for (WS-g-PAA)_{gc} product, 0.754± 0.038 and 0.905±0.026 for (RS-g-PAA)_{gc} product, respectively.

### Characterization of Thiolated Copolymers

- Thiolation of all synthesized S-g-PAA products is realized in this invention. For the aim of comparison, starch and PAA were also thiolated with the same reagent. All of the thiolated products synthesized were obtained as creamy yellow coloured solid substances.
- The structure of the thiolated products as synthesized, was identified by Fourier Transform Infrared Spectroscopy (FTIR) technique. The analysis were performed in the Digilab Excalibur-FTS 3000 MX model FT-IR apparatus. In FTIR analyses, tablets prepared by the dilution in a sample/KBr ratio of 1/200 were used and the spectra were recorded at a wavenumber range of 400-4000 cm⁻¹.

In the thiolation of starch by using Cys, the reaction occurs in between the free methylol groups placing in the glycoside rings in starch molecule, which are activated by EDAC in the beginning and the amine groups in Cys molecule. Accordingly, the new -HC-NH-bonds are formed in the molecular structure of the thiolated products. In the FTIR spectra of the thiolated starch product, a decrease in the intensity of stretching vibration belonging to methylol groups in the starch molecule, approximately at a maximum of 1340 cm⁻¹, on the other hand the appearance of three new small sharp peaks at the maximums of 1412, 1153 and 1080 cm⁻¹ belonging to the stretching vibrations of C-N bond shows that starch can be thiolated with Cys according to the reaction mechanism indicated above.

Similarly, in the FTIR spectra of (RS-g-PAA)_{gc} product and (RS-g-PAA)_{gc}-Cys product obtained from thiolation of the former, the intensity of broad, wide absorption peak related to the vibration of free OH groups (namely, in methylol and carboxyl groups) at about 3000-3200 cm⁻¹ region significantly reduces. Besides this, a sharp absorption peak at a maximum of 3420 cm⁻¹ and the sharp small absorption peak at a maximum of 1551 cm⁻¹belonging to the stretching vibration of the -NH- bonding in the carboxamide structure, and a small absorption peak at a maximum of 1645 cm⁻¹ related to the stretching vibration of C=O (carbonyl) bond in the carboxamide structure appeared. This situation shows that, as expected, new carboxamide bonds were produced in the structure by thiolation reaction occurred on the graft chain in the starch molecule, namely, between the carboxyl group of PAA link (and somewhat the free methylol groups in starch molecule) and the amine group in Cys molecules. In addition to this, in the same spectra a new absorption peak at a maximum of 2552 cm⁻¹ arises in a broad shoulder related to the vibrations of the free SH groups in Cys molecules. This situation also verifies that the thiolation reaction occurred as expected. On the other hand, a new absorption peak in the spectra arises which is at 1220-1270 cm-1 region and at a maximum of around 1248 cm-1. The main reason for this is the sulphur atom places in the molecule not only as a -SH bond but also in different groups in the molecule, for example, in the case if the sulphur atom places in the molecule structure, absorption peak related to the stretching of H2C-S- and/or -C-O-S- bonds arises.

In FTIR spectra belonging to PAA and PAA-Cys, the thiolated products of PAA at the different pH values, the new absorption peaks at a maximum of 3440, 1652, 1655 and 1540 cm⁻¹ arise following the expected reaction between PAA and Cys as mentioned above because of the new carboxamide bonds formed in the molecule.

In the case of PAA-TGA product obtained from thiolation reactions by using TGA, the intensity of the broad, wide absorption peak arising from the free OH group vibrations (namely in the methylol and carboxyl groups) which, at approximately 3000-3200 cm⁻¹ region in the FTIR spectra, reduces significantly similar to the reaction with Cys. In the same spectrum, a new absorption peak at approximately 2672 cm⁻¹ due to the free SH groups stretching in TGA compound appears as a broad shoulder. In addition to this, in the spectra, a new absorption band appears at 1220-1270 cm⁻¹ region at a maximum of around 1244 cm⁻¹. This situation also appears with the reaction products with Cys. As mentioned above, the main reason for this is the sulphur atom places in the molecule not only as a - SH bond but also in different groups in the molecule in the thiolation reactions, for example, in the case if the sulphur atom places in the molecule structure, absorption peak related to the stretching of H₂C-S- and/or -C-O-S- bonds arises.
- The amount of thiol group (µmol/g polymer) of thiolated products was determined by volumetric method (Cevher et al.,2008, AAPS Pharm Sci. Tech., 9(3): 953). The amounts of free thiol group (SH) (µmol/g polymer) were found to be 276.7 for RS-Cys product, 275.6 for (RS-g-PAA)_{gc}-Cys product and 302,17 for (WS-g-PAA)_{gc}-Cys product when Cys was used. It was determined that these values were 8.4 for RS-TGA product, zero (0) for (RS-g-PAA)_{gc}-TGA product and zero (0) for (WS-g-PAA)_{gc}-TGA product when TGA was used. When 2-ITH was used, the values were determined to be 10.5 for RS-ITH product, 8.4 for (RS-g-PAA)_{gc}-ITH product and zero (0) for (WS-g-PAA)_{gc}-ITH product.

When TGA was used as a thiolation reagent in the reactions, the main reason that there were not free thiol group amounts for (RS-g-PAA)_{gc}-TGA and (WS-g-PAA)_{gc}-TGA products is that the assay procedure determines only the free SH groups in the molecule. In other words, if the S enters into molecule not in a SH group but in different bond structures for example in H₂C-S- or -C-O-S- etc., the result will be zero according to this assay procedure. Therefore, as indicated in the descriptions of FTIR spectra in above, in this case, it is observed that the sulphur enters into the structure of the polymer molecule as a -C-S- or -C-O-S- bond but not as a -SH group. Therefore, this situation is also verified by the higher values of work of adhesion for these products.
- In the study, in order to examine the *in vitro* behaviour of all the synthesized products in vagina, the behaviour of the tablets prepared by them in pH 5 lactate buffer was monitored. Depending on the thiolation reagent used in the reactions (Cys, TGA and 2-ITH), when Cys was used, the tablets of RS-Cys and PAA-Cys (synthesized at pH = 4) products were fully disintegrated at the end of 2 hours and the tablet of (RS-g-PAA)_{gc}-Cys product was disintegrated at the end of 24 hours. When TGA was used, the tablets of RS-TGA and PAA-TGA products (synthesized at pH = 4) were fully disintegrated at the end of 2 hours. When 2-ITH was used, the tablet from RS-ITH product was disintegrated at the end of 2 hours.
- In the study, the Qₑ (g H₂O/g product) and ME% values of tablets prepared from the thiolated products in pH = 5 lactate buffer at the end of 24 hours were found to be, depending on the thiolation reactive used in the reactions, for the tablet of (RS-g-PAA)_{gc}-Cys product (at the end of 6 hours) was 8.14 and for the tablet of PAA-Cys product (synthesized at pH = 5) was 106.87 and 52.41%. When TGA was used, the values were determined as 5.82 and 43.13% for the tablet of (RS-g-PAA)_{gc}-TGA product and 6.47 and 36.92% for the tablet of (WS-g-PAA)_{gc}-TGA product. When 2-ITH was used, the values were determined as 18.40 and 12.94 for the tablet of (RS-g-PAA)_{gc}-ITH and 26.98 and 14.62% for PAA-ITH (synthesized at pH = 4), respectively.
- According to the *in vitro* bioadhesion experiments, the work of adhesion (mJ/cm²) and the maximal detachment force (N/cm²) values for tablets prepared from the thiolated products were found to be, depending on the thiolation reagent used, when Cys was used, 1.484±0.076 and 0.569 ± 0.018 for RS-Cys product, 2.323±0.061 and 0.793±0.013 for (RS-g-PAA)_{gc}-Cys product, 1.108±0.022 and 0.491±0.025 for PAA-Cys (synthesized at pH = 4), and 1.251±0.026 and 0.431±0.029 for PAA-Cys (synthesized at pH = 5). When TGA is used, the following values were determined: 0.074±0,016 and 0.100±0,006 for RS-TGA product, 2.027±0.027 and 2.485±0.250 for (WS-g-PAA)_{gc}-TGA product, 5.351±0.457 and 1.394±0.047 for PAA-TGA product (synthesized at pH=4), 6.693±0.091 and 2.209±0,170 for PAA-TGA product (synthesized at pH=5), respectively.

As also indicated in above, when the application of the thiolation reaction, if polymers, besides their other properties, consist of bonds with S atom (namely -C-S-H thiol group and also H₂C-S- and/or C-O-S bond), they get superior mucoadhesive property because of higher interactions of sulphydryl groups (or other groups) with glycoproteins of mucin.

When the free thiol group amount and mucoadhesion properties of (WS-g-PAA)_{gc}-TGA product are evaluated together, in the case of using TGA as thiolation reagent, the work of adhesion/ the maximal detachment force gave the highest value in this group, although the amount of the free thiol group was zero (0). The main reason for this, as indicated in above, that the S atom enters into the structure in H₂C-S- and/or -C-O-S- bonds but not in the free SH group (-C-S-H). Therefore, as indicated in above, the FTIR spectrum of this product also verifies this matter. Accordingly, this structure caused the mucoadhesive property of polymer material to be higher.

### Cytotoxicity Test

In the cytotoxicity test of the synthesized (WS-g-PAA)_{gc} and (WS-g-PAA)_{gc}-TGA copolymers, HEK 293T cells (human embriogenic kidney 293 cells) were used. The polymers were selected in a concentration range of 100-200 ng/ml and the measurements were done by using ACEA Bioscience and Roche RT-CES Technology xCELLigence apparatus for 48 hours in 30 minute intervals. Similarly, the study was also done without using polymer, which was called as "blank". It was proven that the synthesized (WS-g-PAA)_{gc} and (WS-g-PAA)_{gc}-TGA polymers did not have an effect on the cell viability such as cell morphology and cell proliferation even at the highest dose and they can offer the cell viability over around 90%. Accordingly, it was detected that the synthesized starch based copolymers were nontoxic.

### Preparation of the tablets

As presented above, the tablets of different weights were prepared according to the mucoadhesion, swelling and matrix erosion properties using (RS-g-PAA)_{gc}, (WS-g-PAA)_{gc}, (WS-g-PAA)_{g} and (PS-g-PAA)_{gc} products from the synthesized S-g-PAA copolymers and (WS-g-PAA)_{gc}-TGA, (RS-g-PAA)_{gc}-TGA and (WS-g-PAA)_{gc}-Cys products from the thiolated products.
- In the first group, the flat-face rounded tablets with total weight of 300 mg and a diameter of 0.8 cm comprising Tryphan blue in an amount of 0.2% (w/w) and magnesium stearate in an amount of 1% (w/w) of the total weight, were prepared from selected products of starch based copolymers.
- In the second group, the flat-face rounded tablets with total weight of 1.2 g and a diameter of 1.5 cm comprising Tryphan blue in an amount of 0.2% (w/w) and magnesium stearate in an amount of 1% (w/w) of the total weight, were prepared from selected products of starch based copolymers.

### The in vivo "residence time" test of the products in the sheep vagina

One of the success criteria of the study is that it provides with hormone loaded tablets which can stay in the vagina for at least 7-10 days and it also enables to the controlled release of the hormone. Therefore, the *in vivo* residence time test was primarily applied to establish the behaviour of tablets in sheep vagina without using the hormone.

According to the results of this test, the product which remained with the longest time (minimum 7 days) in vagina in a swollen and uniform form was accepted to be successful. The properties which primarily affects the success of the tablet are physical properties of hormone carrier tablet i.e. size, thickness and weight etc. which will be used in the application as well as the swelling, mucoadhesion and matrix adhesion properties. Following this work, the optimization of hormone carrier vaginal tablets was also realized.

It was studied with the tablets of two different weight for more easily and more accurate monitoring the physical changes of the tablets inserted in the sheep vagina, the shape and duration of tablet separating from vulva. In the control of tablets, the following properties were monitored and the proper formulation was established: i.e. whether there was a staining under the tail of the sheep, the physical situation of the tablet inside the vagina and the swelling properties in tablets in certain durations (at 6, 24 and 32 hours and in the following 24 hours).

According to the result of this test, it was established that the tablet remained at the longest time (7.5-8 days) in the vagina, which will be used in the carrying of the hormone was the tablet of 1.2 g weight prepared from the second group from (WS-g-PAA)_{gc} grafting product. As per the thiolated products, the tablet which will be used in the hormone carrying and remained at a longest period (6.5 - 7 days) was found to be the tablet of 1.2 g weight prepared from the second group from (WS-g-PAA)_{gc}-TGA. According to the results from these experiments, tablets of 1.75 g and 1.5 g weight were prepared depending on the dose of progesterone used, provided by keeping the tablet diameter and shape constant.

### Preparation of tablets containing the hormone

In order to investigate the hormone release, the tablets with and without comprising progesterone (in a 1.5 cm diameter, rounded) were prepared.

In the first group, each tablet containing 1.5 g (WS-g-PAA)_{gc} product, magnesium stearate (1 % of total weight) and 50 mg progesterone was prepared by compressing at 5000 psi pressure using a tableting machine.

In the second group, each tablet containing 1.5 g (WS-g-PAA)_{gc} product, magnesium stearate (1% of total weight) and 75 mg progesterone was prepared by compressing at 5000 psi pressure using a tableting machine.

In the third group, each tablet containing 1.75 g of (WS-g-PAA)_{gc} product, magnesium stearate (1% of total weight) and 300 mg progesterone was prepared by compressing at 5000 psi pressure in a tableting machine.

In the fourth group, each tablet containing 1.5 g of (WS-g-PAA)_{gc}-TGA product, magnesium stearate (1% of total weight) and 75 mg of progesterone was prepared by compressing at 5000 psi pressure in a tableting machine.

### In vivo studies

*In vivo studies* were carried out in the periods out of the breeding season for the investigation of the usability of the copolymers which were synthesized in the present study, in the controlled release of progesterone via vaginal route by the aim of estrus synchronization in sheep.
- The species of sheep used were Kivircik and Gokceada species (from Marmara Region), Red Karaman (from East Anatolia Region)
- In order to regress probable corpus luteum, intramuscular injection of prostaglandin PgF2α was applied to ewes at the beginning.
- Before the tablets prepared with the synthesized polymers were inserted into the vagina of the sheep, the blood samples were collected from the sheep. Then the synthesized copolymer based tablets were inserted into the sheep vagina (n=12 sheep/tablets).
- For the purpose of comparison:
   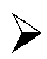 The tablets were also administrated to the ovarioectomized sheep.
   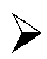 The commercial vaginal product (Chronogest CR-Intervet-Turkey) of 40x30 mm in size, cylindrical polyurethane sponge impregnated 20 mg cronolone (flugestone acetate/florogestone acetate) which is a synthetic progesterone compound was placed into the sheep vagina.
   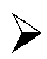 The commercial product which was obtained from the market, the Pfizer Product EAZI-BREED CIDR Sheep and Goat Device comprising 330 mg progesterone was placed into the sheep vagina.
   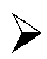 The blood samples were also collected from the sheep named as "blank" which was not treated at all.
- Following the placement of the prepared tablets into vagina, the blood samples were collected at 30 minutes, 3rd, 6th, 8th and 24th hours and then in every 24 hours for 17 days and the serum was separated and these samples were kept in - 20°C.
- For the progesterone assay in the serum of the sheep, in which the tablets containing progesterone were placed, the measurements were taken using Progesteron RIA kit (Radioimmunoassay for the *in vitro* determination of progesterone in human serum and plasma, Immunotech RIA PROGESTERON, Beckman Coulter Company, France; analytical sensitivity 0,05ng/mL, measurement range 0,05-50 ng/mL) in Ria Gamma Counter apparatus (The Genesys Genii Series Multi-Well Gamma Counters, Laboratory Technologies Inc. USA).

The hormone release profiles which were obtained by *in vivo* applying the tablets containing progesterone prepared from the synthesized (WS-g-PAA)_{gc} and (WS-g-PAA)_{gc}-TGA products are respectively given in Figures 1-3, comparatively with the commercial products.

In estrus synchronization, the level of progesterone must be > 1 ng/ml in about 1 day after the dosage form is applied into vagina. In addition, this value must be in between 1 and 5 ng/ml for about 12 days and this is required to be < 0.5 ng/ml at the end of this period.

According to *in vivo* working results, in the commercial sponge applied sheep (which is often used in estrus synchronization), it was observed that the progesterone levels in the blood were kept about 1.5 ng/ml for 8 days; at the 10th day, it increases to about 5.8 ng/ml and from 11th day, it rapidly decreases to about 1.5 ng/ml (Figure 3). In the CIDR applied sheep, it was observed that the release was in the desired amount (higher than a value of 1 ng/ml in between 1 and 12 day) (Figure 3).

In the studies with the tablets (1.5g) prepared with (WS-g-PAA)_{gc} containing 50 mg progesterone, ovarioectomised sheep were also used for the purpose of preventing the release of endogenous progesterone and for not obtaining misleading results. As seen in Figure 1, it was observed that the hormone release was in a high level in the first day (burst effect), but it declined from the second day and it decreased to the low level by declining under the effective level, and it was seen that the tablet kept the blood progesterone hormone levels in the desired levels at gradually increasing concentrations by releasing of the hormone again from approximately 6th day. It was observed that the desired hormone levels (approximately 2 ng/ml) were able to reach only in a 14-16th days term (very long duration) because of the low amount of progesterone in the tablet.

The hormone release of tablets prepared from (WS-g-PAA)gc product containing 75 mg progesterone is extremely over the desired value of 1 ng/ml from the 1st day and measured as 4.4 ng/ml (Figures 2 and 3). It is seen that the hormone release continues from the 3rd day in a controlled way without declining above the value of 1 ng/ml for the following 14 days. From the 16th day, the curve starts slightly to rise (Figure 1). Consequently, the hormone release in the blood of the sheep which are administrated with 75 mg progesterone containing tablet has a similar behaviour with those administrated with 300 mg progesterone (a higher amount) containing commercial product CIDR. Apart from that it has shown an immediate release (burst effect) similar to this commercial product, from the beginning of the application within first 24 hours (Figure 2).

However, in case of the tablets prepared from (WS-g-PAA)_{gc}-TGA product containing 75 mg progesterone, the hormone release in blood from the 1st day is above the desired 1 ng/ml value (Figures 2 and 3). During the following 14 day, the hormone release is continued in a controlled manner without declining below the 1 ng/ml value. However, from 15th day and the following 16th and 17th days, this figure is at 0.9, 0.5 and 0.5 ng/ml values respectively (Figure 3).

As a result of this, in the vaginal administration for the release of progesterone;
- The starch based modified copolymers can be used as non-toxic, biocompatible and mucoadhesive drug carrier polymer.
- Mucoadhesive vaginal tablet with 75 mg progesterone containing (WS-g-PAA)_{gc} graft copolymer carrier in the tablet formulations and mucoadhesive vaginal tablet with 75 mg progesterone containing (WS-g-PAA)_{gc}-TGA thiolated graft copolymer carrier can be used as an alternative products to 20 mg progestagen (cronolone; flugestone acetate/florogestone acetate) containing sponge and 330 mg progesterone containing CIDR which are often and conventionally used in estrus synchronization.

## Claims

1. Use of starch-graft-polyacrylic acid copolymers or thiolated forms thereof in manufacturing of an extended release vaginal drug carrier system.

2. The use according to Claim 1 wherein the said starch-graft-polyacrylic acid copolymer is originated of a starch selected from those of wheat, rice, potato or maize.

3. The use according to Claim 2 wherein said starch-graft-polyacrylic acid copolymer is originated of wheat starch.

4. The use according to Claim 1 wherein the carrier system comprises a local or systemically effective drug for vaginal administration.

5. The use according to any of the preceding claims wherein the carrier system comprises a hormone for providing estrus synchronization in veterinary medicine.

6. The use according to Claim 5 wherein said hormone is progesterone.

7. The use according to Claim 6 wherein dosage of the hormone is between 50 mg and 300 mg.

8. The use according to any of the preceding claims wherein dosage form of the carrier system is selected from the group consisting of tablet, pessary, ovule, vaginal suppository and gel form.

9. The use according to Claim 8 wherein the carrier system is in the form a tablet.

10. The use according to claim 1 wherein graft amount (GA%) of the starch-graft-poly(acrylic acid) copolymer is between 21.32±0.5 (wt.%) and 24.84±2.0 (wt.%).

11. The use according to claim 1 wherein thiol group amount of the thiolated forms of the starch-graft-poly(acrylic acid) copolymer is between 8 and 305 µmol/g polymer.

12. The use according to claim 1 wherein the thiolated form is a thiolated starch-graft-polyacrylic acid copolymer which is thiolated on graft chain of the starch molecule.

13. The use according to claim 12 wherein the thiolated starch-graft-polyacrylic acid copolymer is prepared by a method comprising reaction of a starch compound with an acrylic acid monomer, and thiolating the starch-graft-polyacrylic acid copolymer with a compound selected from the group consisting of L-cysteine hydrochloride monohydrate, thioglycolic acid and 2-iminothiolane hydrochloride.

14. Use of a composition comprising a starch-graft-polyacrylic acid copolymer or thiolated forms thereof and a progesterone hormone for estrus synchronization in animals by vaginal route.

15. The use according to claim 14 wherein the composition is in a dosage form selected from the group of tablet, pessary, ovule, vaginal suppository and gel forms.

## Patentansprüche

1. Verwendung von Stärke-pfropf-Polyacrylsäure-Copolymeren oder von thiolierten Formen davon bei der Herstellung eines vaginalen Wirkstoffträgersystems mit verlängerter Wirkstofffreisetzung.

2. Verwendung gemäß Anspruch 1, wobei das Stärke-pfropf-Polyacrylsäure-Copolymer auf eine Stärke zurückgeht, die aus Weizen-, Reis-, Kartoffel- oder Maisstärke ausgewählt ist.

3. Verwendung gemäß Anspruch 2, wobei das Stärke-pfropf-Polyacrylsäure-Copolymer auf Weizenstärke zurückgeht.

4. Verwendung gemäß Anspruch 1, wobei das Trägersystem einen lokal oder systemisch wirksamen Wirkstoff für die vaginale Verabreichung umfasst.

5. Verwendung gemäß einem der vorstehenden Ansprüche, wobei das Trägersystem ein Hormon umfasst, um in der Tiermedizin für eine Synchronisierung der Brunst zu sorgen.

6. Verwendung gemäß Anspruch 5, wobei es sich bei dem Hormon um Progesteron handelt.

7. Verwendung gemäß Anspruch 6, wobei die Dosierung des Hormons 50 mg bis 300 mg beträgt.

8. Verwendung gemäß einem der vorstehenden Ansprüche, wobei die Darreichungsform des Trägersystems aus der Gruppe ausgewählt ist, die aus einer Tablette, einem Pessar, einer Ovula, einem Vaginalzäpfchen und einer Gelform besteht.

9. Verwendung gemäß Anspruch 8, wobei das Trägersystem in Form einer Tablette vorliegt.

10. Verwendung gemäß Anspruch 1, wobei die Pfropfmenge (GA%) des Stärke-pfropf-Polyacrylsäure-Copolymers zwischen 21,32 ± 0,5 (Gew.-%) und 24,84 ± 2,0 (Gew.-%) liegt.

11. Verwendung gemäß Anspruch 1, wobei die Menge der Thiolgruppen der thiolierten Formen des Stärke-pfropf-Polyacrylsäure-Copolymers zwischen 8 und 305 µmol/g Polymer liegt.

12. Verwendung gemäß Anspruch 1, wobei die thiolierte Form ein thioliertes Stärke-pfropf-Polyacrylsäure-Copolymer ist, das an der Pfropfkette des Stärkemoleküls thioliert ist.

13. Verwendung gemäß Anspruch 12, wobei das thiolierte Stärke-pfropf-Polyacrylsäure-Copolymer nach einem Verfahren hergestellt ist, das das Umsetzen einer Stärkeverbindung mit einem Acrylsäuremonomer und das Thiolieren des Stärke-pfropf-Polyacrylsäure-Copolymers mit einer Verbindung, die aus der Gruppe ausgewählt ist, welche aus L-Cystein-Hydrochlorid-Monohydrat, Thioglycolsäure und 2-Iminothiolan-Hydrochlorid besteht, umfasst.

14. Verwendung einer Zusammensetzung, die ein Stärke-pfropf-Polyacrylsäure-Copolymer oder thiolierte Formen davon und ein Progesteronhormon umfasst, zur Synchronisierung der Brunst bei Tieren auf dem vaginalen Weg.

15. Verwendung gemäß Anspruch 14, wobei die Zusammensetzung in einer Darreichungsform vorliegt, die aus der Gruppe Tablette, Pessar, Ovula, Vaginalzäpfchen und Gelformen ausgewählt ist.

## Revendications

1. Utilisation de copolymères d'acide polyacrylique greffé sur amidon ou de formes thiolées en dérivant, dans la fabrication d'un système de support de médicament vaginal à libération prolongée.

2. L'utilisation selon la revendication 1, dans laquelle ledit copolymère d'acide polyacrylique greffé sur amidon provient d'un amidon choisi parmi ceux du blé, du riz, de la pomme de terre ou du maïs.

3. L'utilisation selon la revendication 2, dans laquelle ledit copolymère d'acide polyacrylique greffé sur amidon provient d'amidon de blé.

4. L'utilisation selon la revendication 1, dans laquelle le système de support comprend un médicament local ou systémiquement efficace pour l'administration vaginale.

5. L'utilisation selon l'une quelconque des revendications précédentes, dans laquelle le système de support comprend une hormone pour fournir une synchronisation de l'oestrus en médecine vétérinaire.

6. L'utilisation selon la revendication 5, dans laquelle ladite hormone est la progestérone.

7. L'utilisation selon la revendication 6, dans laquelle le dosage de l'hormone est compris entre 50 mg et 300 mg.

8. L'utilisation selon l'une quelconque des revendications précédentes, dans laquelle la forme posologique du système de support est choisie dans le groupe consistant en un comprimé, un pessaire, un ovule, un suppositoire vaginal et un gel.

9. L'utilisation selon la revendication 8, dans laquelle le système de support est sous la forme d'un comprimé.

10. L'utilisation selon la revendication 1, dans laquelle la quantité de greffage (GA%) du copolymère d'acide polyacrylique greffé sur amidon est comprise entre 21,32 ± 0,5 (% en poids) et 24,84 ± 2,0 (% en poids).

11. L'utilisation selon la revendication 1, dans laquelle la quantité de groupes thiol des formes thiolées du copolymère d'acide polyacrylique greffé sur amidon est comprise entre 8 et 305 µmol/g de polymère.

12. L'utilisation selon la revendication 1, dans laquelle la forme thiolée est un copolymère thiolé d'acide polyacrylique greffé sur amidon qui est thiolé sur la chaîne de greffe de la molécule d'amidon.

13. L'utilisation selon la revendication 12, dans laquelle le copolymère thiolé d'acide polyacrylique greffé sur amidon est préparé par un procédé comprenant la réaction d'un composé d'amidon avec un monomère consistant en un acide acrylique et par la thiolation du copolymère d'acide polyacrylique greffé sur amidon avec un composé choisi dans le groupe consistant en un chlorhydrate de L-cystéine monohydraté, un acide thioglycolique et un chlorhydrate de 2-iminothiolane.

14. Utilisation d'une composition comprenant un copolymère d'acide polyacrylique greffé sur amidon ou des formes thiolées en dérivant et une hormone progestérone pour la synchronisation de l'oestrus par voie vaginale chez les animaux.

15. L'utilisation selon la revendication 14, dans laquelle la composition est sous une forme posologique choisie dans le groupe des formes de comprimé, de pessaire, d'ovule, de suppositoire vaginal et de gel.
